# EUROPÄISCHE PATENTSCHRIFT

(11) **EP 0 829 465 B1**
(45) Veröffentlichungstag und Bekanntmachung des Hinweises auf die Patenterteilung: **23.08.2000**
(21) Anmeldenummer: 97115613.8
(22) Anmeldetag: 09.09.1997
(51) Int. Cl.: C07C 49/675, C07C 45/46

(54) **Verfahren zur Herstellung substituierter Indanone**
Process for the preparation of substituted indanones
Procédé pour la préparation d'indanones substituées

(30) Priorität: 12.09.1996 DE 19637128
(43) Veröffentlichungstag der Anmeldung: 18.03.1998
(73) Patentinhaber: BASF AKTIENGESELLSCHAFT, 67056 Ludwigshafen (DE)
(72) Erfinder: Horn, Hans Christoph, Dr., 67245 Lambsheim (DE); Spahl, Roland, Dr., 64653 Lorsch (DE); Müller, Hans-Joachim, Dr., 67269 Grünstadt (DE); Trübenbach, Peter, Dr., 67065 Ludwigshafen (DE); Rieger, Bernhard, Prof. Dr., 89075 Ulm (DE); Wagner, Jürgen Matthäus, 89160 Dornstadt (DE); Dietrich, Ulf, 89079 Ulm (DE)

(56) Entgegenhaltungen:
- EP-A- 0 089 302
- EP-A- 0 587 107
- FR-A- 631 003
- PATENT ABSTRACTS OF JAPAN vol. 096, no. 005, 31.Mai 1996 & JP 08 012615 A (MITSUI PETROCHEM IND LTD), 16.Januar 1996,
- R.T. HART ET AL.: "Acylation-Alkylation Studies" JOURNAL OF THE AMERICAN CHEMICAL SOCIETY., Bd. 72, 1950, DC US, Seiten 3286-3287, XP002049168

## Beschreibung

Gegenstand der vorliegenden Erfindung ist ein Verfahren zur Herstellung von Verbindungen der allgemeinen Formeln Ia und Ib 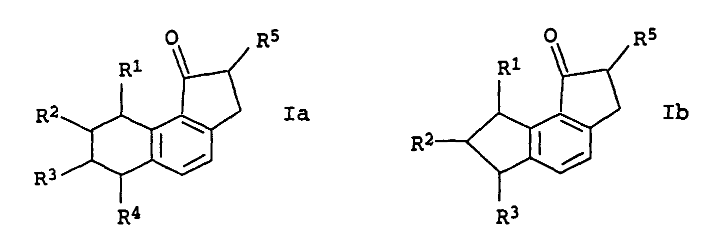 worin R¹, R², R³ und R⁴ Wasserstoff, C₁-C₄-Alkyl, C₁-C₄-Fluoralkyl, Silyl, C₁-C₄-Alkylsilyl, Phenyl oder Benzyl und R² und R³ auch gemeinsam mit den Kohlenstoffatomen, an welche sie gebunden sind, einen Benzolring und R⁵ einen C₁-C₁₀-Alkylrest, einen Phenylrest oder einen C₁-C₄-alkylsubstituierten Phenylrest bedeuten.

Indanone der allgemeinen Formeln Ia und Ib sind wichtige Vorstufen für Metallocenkomplexe, die als Polymerisationskatalysatoren Verwendung finden. Dazu werden die Indanone reduziert und dehydratisiert; die resultierenden Indenderivate können mit Übergangsmetallen zu den entsprechenden Metallocenkomplexen umgesetzt werden.

Zur Herstellung substituierter Indanone sind verschiedene Verfahren bekannt. In der EP-A1-549 900 wird die Synthese eines substituierten Benzindanons beschrieben. Der Fünf ring wird dabei in einer mehrstufigen Synthese durch Umsetzung mit einem Malonester, alkalische Verseifung des Diesters, thermische Decarboxylierung, Chlorierung der verbleibenden Carboxylgruppe und intramolekulare Friedel-Crafts-Acylierung gebildet. Die Synthese ist durch ihre Vielstufigkeit mit großem technischen Aufwand verbunden.

In der EP-A1-567 953 wird eine Synthese von Indanonderivaten beschrieben, wobei der Fünf ring durch Umsetzung eines entsprechenden Benzolderivates mit einem substituierten Acrylsäureester in flüssigem Fluorwasserstoff gebildet wird. EP-A1-587 107 beschreibt die Herstellung von 2-Methyl-benzindanon durch Umsetzung von Naphthalin mit Methacrylsäureanhydrid in Gegenwart von BF₃/Fluorwasserstoff. Auch diese Synthesewege sind wegen der schwierigen Handhabung der äußerst toxischen Flußsäure mit erheblichem technischen Aufwand verbunden.

Ein weiterer Syntheseweg für Indanone wird in der EP-A1-545 304 beschrieben. Die Herstellung erfolgt hier durch Umsetzung eines Benzolderivates mit α-Halogenalkylpropionsäurehalogeniden, bevorzugt mit α-Bromisobuttersäurebromid. Nachteilig an dieser Synthese ist der große Anfall halogenhaltiger Abfälle.

Der vorliegenden Erfindung lag daher die Aufgabe zugrunde, einen einfachen Syntheseweg zur Herstellung von Benzindanonen der allgemeinen Formel I zu finden, der die Nachteile der bekannten Synthesewege überwindet.

Demgemäß wurde ein Verfahren zur Herstellung von Verbindungen der allgemeinen Formeln Ia und Ib 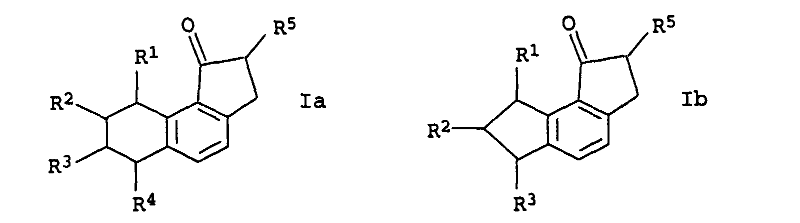 worin R¹, R², R³ und R⁴ Wasserstoff, C₁-C₄-Alkyl, C₁-C₄-Fluoralkyl, Silyl, C₁-C₄-Alkylsilyl, Phenyl oder Benzyl und R² und R³ auch gemeinsam mit den Kohlenstoffatomen, an welche sie gebunden sind, einen Benzolring und R⁵ einen C₁-C₁₀-Alkylrest, einen Phenylrest oder einen C₁-C₄-alkylsubstituierten Phenylrest bedeuten, gefunden, welches dadurch gekennzeichnet ist, daß man Verbindungen IIa bzw. IIb 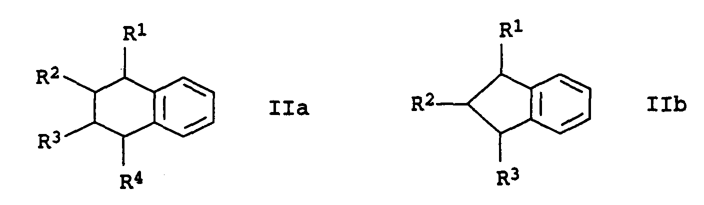 mit einer Verbindung der Formel III 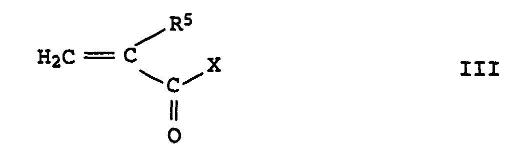 in welcher X Chlor, Brom oder Jod bedeutet, in Gegenwart eines Friedel-Crafts-Katalysators in einem Schritt zu Verbindungen Ia bzw. Ib umsetzt.

Bei der Umsetzung der Verbindungen IIa bzw. IIb mit einer Verbindung III können verschiedene Isomere entstehen. Das erfindungsgemäße Verfahren weist jedoch eine ausgeprägte Stereoselektivität auf, so daß meist nur die Isomeren Ia bzw. Ib beobachtet wurden.

In den Indanonderivaten Ia und Ib, die nach dem erfindungsgemäßen Verfahren herstellbar sind, können die Substituenten R¹, R², R³ und R⁴ neben Wasserstoff beispielsweise Methyl, Ethyl, Isopropyl, n-Propyl, n-Butyl, Isobutyl, sec-Butyl oder tert-Butyl bedeuten, wobei Wasserstoff besonders bevorzugt ist. Weiterhin können die Reste R¹ bis R⁴ C₁-C₄-Fluoralkylgruppen bedeuten, also beispielsweise die oben genannten Alkylgruppen, bei denen ein oder mehrere oder auch alle Wasserstoffatome gegen Fluor ausgetauscht sind. Auch Silyl- oder C₁-C₄-Alkylsilylgruppen kommen als R¹ bis R⁴ in Betracht, wobei auch diese Alkylsilylgruppen die oben genannten Alkylgruppen enthalten können. Weiterhin sind bevorzugte Verbindungen Ia solche, in denen R² und R³ gemeinsam mit den Kohlenstoffatomen, an welche sie gebunden sind, einen Benzolring bilden. Dieser Benzolring kann wiederum einfach, doppelt, dreifach oder vierfach mit Resten der Bedeutung von R¹ bis R⁴ substituiert sein.

Der Substituent R⁵ wird über die Verbindung III in die Zielverbindungen eingeführt. R⁵ ist vorzugsweise Methyl oder Ethyl, besonders bevorzugt Methyl, jedoch kommen auch andere C₁-C₁₀-Alkylreste, der Phenylrest oder C₁-C₄-alkylsubstituierte Phenylreste in Betracht. Als C₁-C₁₀-Alkylreste kommen neben den obengenannten C₁-C₄-Alkylresten auch die verschiedenen Isomeren Pentyl-, Hexyl-, Heptyl-, Octyl-, Nonyl- und Decylreste in Betracht. Als Phenylreste kommen neben dem bevorzugten unsubstituierten Phenylrest besonders einfach mit Methyl oder Ethyl substituierte Phenylreste in Betracht.

Der Rest X in der Verbindung III kann Chlor, Brom oder Iod bedeuten, bevorzugt ist X Chlor.

Das erfindungsgemäße Verfahren nutzt als Katalysator einen Friedel-Crafts-Katalysator. Als Friedel-Crafts-Katalysatoren kommen alle üblichen derartigen Katalysatoren in Frage, beispielsweise AlCl₃, AlBr₃, BF₃, ZnCl₂, FeCl₃, SnCl₄, TiCl₄, SiCl₄, PCl₅ und SbCl₅, vorzugsweise wird AlCl₃ verwendet.

Der Friedel-Crafts-Katalysator, insbesondere AlCl₃, wird im allgemeinen im Überschuß eingesetzt, vorzugsweise in einer Menge, daß das Molverhältnis von Katalysator zur Verbindung III zwischen 1 und 2, besonders bevorzugt zwischen 1 und 1,5, ganz besonders bevorzugt zwischen 1,05 und 1,3 beträgt.

Für das erfindungsgemäße Verfahren sind alle üblichen Friedel-Crafts-Lösungsmittel geeignet, wobei Schwefelkohlenstoff, z.B. wegen seiner Toxizität, weniger geeignet ist. In Betracht kommen besonders chlorierte Kohlenwasserstoffe wie Methylenchlorid, 1,2-Dichlorethan, 1,1,2,2-Tetrachlorethan und Chlorbenzol, besonders bevorzugt dient Methylenchlorid als Lösungsmittel.

Die Umsetzung wird bevorzugt bei niedrigen Temperaturen zwischen -80 und 0°C durchgeführt, besonders bevorzugt zwischen -20 und -10°C. Zum Ende der Umsetzung kann zur Vervollständigung der Ringschlußreaktion die Temperatur jedoch angehoben werden, z.B. auf 10-40°C, vorzugsweise auf 20-30°C.

Der Druck hat im allgemeinen keinen nennenswerten Einfluß auf die Reaktion, so daß üblicherweise bei Normaldruck gearbeitet wird.

Das erfindungsgemäße Verfahren zeichnet sich durch einfache technische Handhabung (Einstufenverfahren) und geringen Anfall halogenhaltiger Abfälle aus. Zugleich weist es eine ausgezeichnete Stereoselektivität auf und liefert die gewünschten Produkte in sehr guten Ausbeuten.

Das folgende Beispiel erläutert die Erfindung:

### Beispiel

Herstellung von 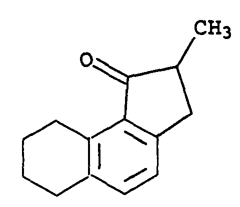 22,2 ml Methacrylsäurechlorid (228,7 mmol) wurden zusammen mit 61 g (457,4 mmol) wasserfreiem Aluminiumchlorid in 250 ml CH₂Cl₂ aufgenommen, auf -78°C gekühlt und langsam mit 31,2 ml Tetrahydronaphthalin (Tetralin, 30,24 g, 228,7 mmol) versetzt. Während der Zugabe des Tetralins erfolgte ein Farbumschlag von hellgelb nach orange. Die Mischung wurde vorsichtig mit wenig verd. HCl versetzt, um ausgefallenes Aluminiumchloridgel in Lösung zu bringen, mit wäßriger K₂CO₃-Lösung und Wasser gewaschen, über Na₂SO₄ getrocknet und anschließend umkristallisiert.
Ausbeute 35,3 g (176,5 mmol) kristallines Produkt, 77,7 % d. Th.
NMR (200 MHz, CDCl₃ 7,24 ppm): ö 1,25 ppm d (J = 7,5 Hz) 3 H Methylgruppe, δ 1,84 ppm m (J = 2-3 Hz) 4 H aliphatische Protonen der Cyclen, δ 2,48 ppm dd (J = 2,5 und 17 Hz) 1 H alkphatisches Proton, δ 2,65 ppm m (J = 2-4 Hz) 3 H aliphatische Protonen der Cyclen, δ 2,74 ppm m (J = 4-5 Hz) 2 H aliphatische Protonen des Indanonsystems, δ 3,2 ppm q (J = 7,5 Hz) 1 H Methinproton Indanonsystem, δ 7,05 und 7,7 ppm d (J = 8 Hz) 2 H aromatisch.
Massenspektrum (FD): m/z 200 (M⁺ 100 %).

## Patentansprüche

1. Verfahren zur Herstellung von Verbindungen der allgemeinen Formeln Ia und Ib 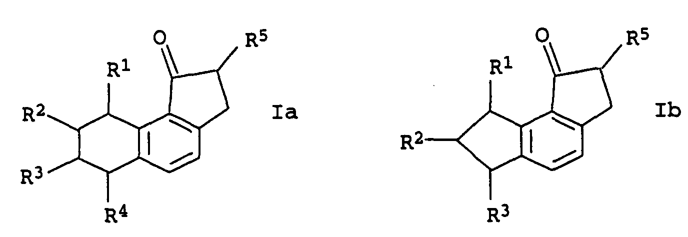 worin R¹, R², R³ und R⁴ Wasserstoff, C₁-C₄-Alkyl, C₁-C₄-Fluoralkyl, Silyl, C₁-C₄-Alkylsilyl, Phenyl oder Benzyl und R² und R³ auch gemeinsam mit den Kohlenstoffatomen, an welche sie gebunden sind, einen Benzolring und R⁵ einen C₁-C₁₀-Alkylrest, einen Phenylrest oder einen C₁-C₄-alkylsubstituierten Phenylrest bedeuten, dadurch gekennzeichnet, daß man Verbindungen IIa bzw. IIb 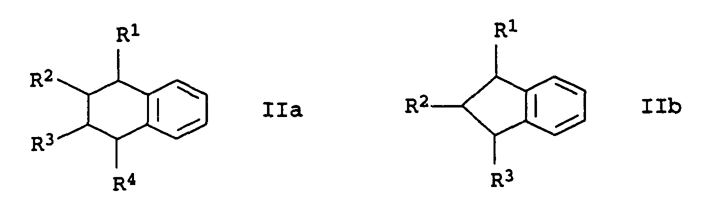 mit einer Verbindung der Formel III 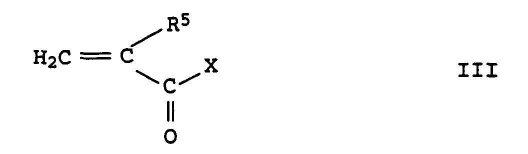 in welcher X Chlor, Brom oder Iod bedeutet, in Gegenwart eines Friedel-Crafts-Katalysators in einem Schritt zu einer Verbindung Ia bzw. Ib umsetzt.

2. Verfahren nach Anspruch 1, dadurch gekennzeichnet, daß R¹, R², R³ und R⁴ Wasserstoff bedeuten.

3. Verfahren nach den Ansprüchen 1 und 2, dadurch gekennzeichnet, daß R⁵ Methyl oder Ethyl bedeutet.

4. Verfahren nach den Ansprüchen 1 bis 3, dadurch gekennzeichnet, daß als Friedel-Crafts-Katalysator AlCl₃ eingesetzt wird.

5. Verfahren nach den Ansprüchen 1 bis 4, dadurch gekennzeichnet, daß das Molverhältnis von AlCl₃ zur Verbindung III 1,0 bis 1,5 beträgt.

6. Verfahren nach den Ansprüchen 1 bis 5, dadurch gekennzeichnet, daß die Umsetzung in Gegenwart von Methylenchlorid durchgeführt wird.

7. Verfahren nach den Ansprüchen 1 bis 6, dadurch gekennzeichnet, daß die Umsetzung bei Temperaturen zwischen -80 und 0°C durchgeführt wird.

## Claims

1. A process for preparing compounds of the general formulae Ia and Ib 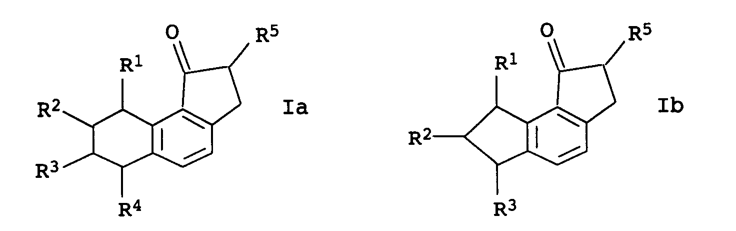 where R¹, R², R³ and R⁴ are each hydrogen, C₁-C₄-alkyl, C₁-C₄-fluoroalkyl, silyl, C₁-C₄-alkylsilyl, phenyl or benzyl and R² and R³ may also form a benzene ring together with the carbons that they are attached to and R⁵ is C₁-C₁₀-alkyl, phenyl or C₁-C₄-alkyl-substituted phenyl, which comprises reacting the compounds IIa or IIb 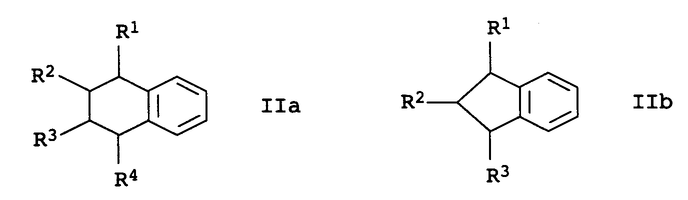 with a compound of the formula III 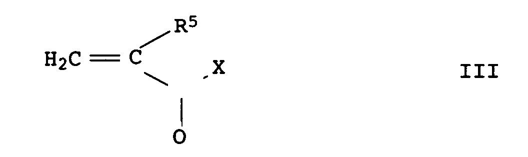 where X is chlorine, bromine or iodine in the presence of a Friedel-Crafts catalyst in one step to give a compound Ia or Ib.

2. A process as claimed in claim 1, wherein R¹, R², R³ and R⁴ are each hydrogen.

3. A process as claimed in claim 1 or 2, wherein R⁵ is methyl or ethyl.

4. A process as claimed in any of claims 1 to 3, wherein the Friedel-Crafts catalyst used is AlCl₃.

5. A process as claimed in any of claims 1 to 4, wherein the molar ratio of AlCl₃ to the compound III is from 1.0:1 to 1.5:1.

6. A process as claimed in any of claims 1 to 5, wherein the reaction is carried out in the presence of methylene chloride.

7. A process as claimed in any of claims 1 to 6, wherein the reaction is carried out at from -80 to 0°C.

## Revendications

1. Procédé de préparation de composés des formules générales la et Ib: 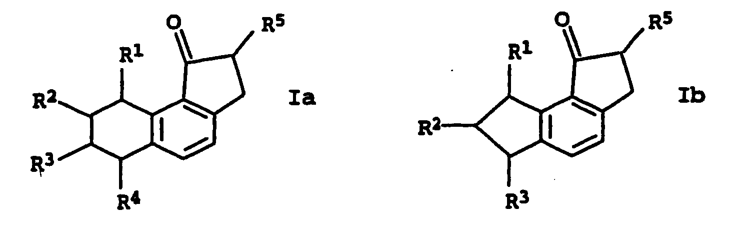 dans lesquelles R¹, R², R³ et R⁴ représentent de l'hydrogène, un groupe alkyle en C₁-C₄, fluoroalkyle en C₁-C₄, silyle, alkylsilyle en C₁-C₄, phényle ou benzyle et R² et R³ peuvent aussi former, conjointement avec les atomes de carbone auxquels ils sont liés, un noyau de benzène, et R⁵ représente un radical alkyle en C₁-C₁₀, un radical phényle ou un radical phényle substitué par de l'alkyle en C₁-C₄, caractérisé en ce qu'on fait réagir des composés des formules lla ou respectivement IIb: 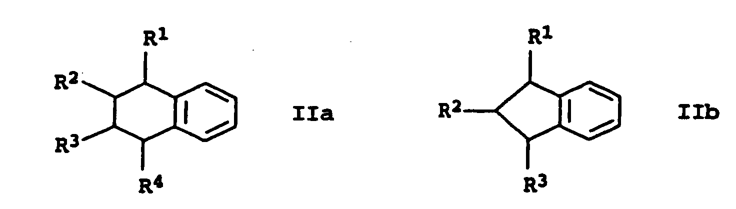 avec un composé de la formule III : 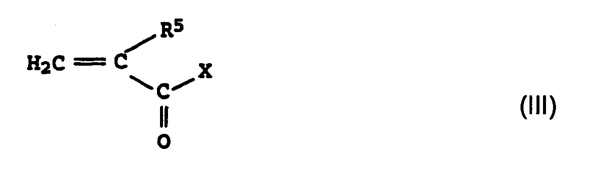 dans laquelle X représente du chlore, du brome ou de l'iode, en présence d'un catalyseur de Friedel-Crafts en une étape pour former un composé de formule la ou respectivement Ib.

2. Procédé suivant la revendication 1, caractérisé en ce que R¹, R², R³ et R⁴ représentent de l'hydrogène.

3. Procédé suivant l'une des revendications 1 et 2, caractérisé en ce que R⁵ représente du méthyle ou de l'éthyle.

4. Procédé suivant l'une des revendications 1 à 3, caractérisé en ce que, comme catalyseur de Friedel-Crafts, on met en oeuvre AlCl₃.

5. Procédé suivant l'une des revendications 1 à 4, caractérisé en ce que le rapport molaire entre AlCl₃ et le composé III est de 1,0 à 1,5.

6. Procédé suivant l'une des revendications 1 à 5, caractérisé en ce que la réaction est effectuée en présence de chlorure de méthylène.

7. Procédé suivant l'une des revendications 1 à 6, caractérisé en ce que la réaction est effectuée à des températures comprises entre -80 et 0°C.
